# EUROPEAN PATENT SPECIFICATION

(11) **EP 1 601 338 B1**
(45) Date of publication and mention of the grant of the patent: **06.07.2011**
(21) Application number: 04714939.8
(22) Date of filing: 26.02.2004
(51) Int. Cl.: A61K 8/36, A61K 8/365, A61Q 15/00, A61K 8/368

(54) **PSEUDO BODY ODOR COMPOSITION**
PSEUDOKÖRPERGERUCHSZUSAMMENSETZUNG
PREPARATION AYANT UNE PSEUDO ODEUR CORPORELLE

(30) Priority: 03.03.2003 JP 2003056017; 04.03.2003 JP 2003057462
(43) Date of publication of application: 07.12.2005
(60) Divisional application: 10177437.0
(73) Proprietor: Takasago International Corporation, Tokyo-to 144-8721 (JP)
(72) Inventor: Ogura, Miharu, c/o Takasago Int. Corporation, Hiratsuka-shi, Kanagawa 2540073 (JP); Sakurai, Kazutoshi, c/o Takasago Int. Corporation, Tokyo 1448721 (JP); Sawano, Kiyohito, c/o Takasago Int. Corporation, Hiratsuka-shi, Kanagawa 2540073 (JP); Yamazaki, Sadahiko, c/o Takasago Int. Corporation, Hiratsuka-shi, Kanagawa 2540073 (JP); Hirano, Koji, c/o Takasago Int. Corporation, Hiratsuka-shi, Kanagawa 2540073 (JP)
(74) Representative: Neidl-Stippler, Cornelia
(86) International application number: PCT/JP2004/002300
(87) International publication number: WO 2004/078154

(56) References cited:
- WO-A-93/07853
- WO-A-02/085294
- WO-A-03/052411
- US-A- 5 141 921
- PATENT ABSTRACTS OF JAPAN vol. 1997, no. 08, 29 August 1997 (1997-08-29) & JP 9 104892 A (KANEBO LTD), 22 April 1997 (1997-04-22)
- PATENT ABSTRACTS OF JAPAN vol. 1998, no. 05, 30 April 1998 (1998-04-30) & JP 10 025265 A (KAO CORP), 27 January 1998 (1998-01-27)

## Description

### Technical Field

The present invention relates to a pseudo body odor composition more close to a peculiar odor that an actual body odor has, in more detail, a pseudo body odor composition that is more close to a peculiar odor that the body odor has such as an odor of sweat and an odor of armpit due to a human body and can be preferably used for evaluating an effect of masking, harmonizing or deodorizing the odor of sweat, the odor of armpit and so on ; and a method of evaluating the effect of masking, harmonizing or deodorizing the odor of sweat, the odor of armpit and so on by using this pseudo body odor composition.

### Background Art

From a rise in the sense of cleanliness, care for, in particular, odors has become very important recently; among these, needs for eliminating odors emitted from human bodies, that is, body odors are particularly high. As the odors emitted from a human body, a scalp or head hair odor, a mouth odor, a sweat odor, an armpit odor, a foot odor and so on are known. On the other hand, there are also high demands for deodorizing a mouth odor and an excrement and urine odor due to sulfur compounds and so on, and a putrefaction odor due to particular components of decomposition products of organic compounds. Among these, the armpit odor is considered that the odor is generated by that secretions from sweat glands such as a sebaceous gland, an apocrine gland, an eccrine gland and so on are oxidized or subjected to an action of microbes. The odors such as the sweat odor, the foot odor and so on including the armpit odor generate unpleasant sensations in persons. Accordingly, there have so far been various devices to eliminate the odor. In view of these circumstances, products that intend to inhibit or reduce the body odors including the armpit odor and the sweat odor have been much provided to the market.

As methods of eliminating the odor, there have been known various methods such as a method of inhibiting the generation of the odor by inhibiting the generation of substances that generate odor materials, a method of inhibiting proliferation of microbes relevant to odor generation, and a method of masking an odor material with a particular perfume. In various kinds of commercially available products, at least any one of these methods for eliminating the odor is adopted. For illustration purpose, for instance, an antiperspirant that suppresses an amount of sweat that generates an odor material, a bacteriostat or microbicide that restrains proliferation of microbes, a masking agent due to a scent-emitting component and so on are compounded into commercial products in accordance with the object. In the antiperspirants, in almost all, an aluminum compound that is an astringent is used as a main component, usually aluminum chloride being used. Further, as the bacteriostat or microbicide, hexachlorophene and various kinds of quaternary ammonium chloride compounds are generally used. Furthermore, as the masking agent due to a scent-emitting component, there have been used various kinds of perfumes such as described in Japanese Patent Application Laid-open (JP-A) No. 6-179610, Japanese Patent Application National Publication (Laid-Open) No. 2002-505264, and Japanese Patent Office: Published collection of well-known prior arts (perfume), part **I,** general of perfume (Jan. 29,1999) pp. 230 to 250.

When various kinds of products that have the masking or deodorizing effect to such odors are developed, it needs evaluating previously the masking effect and deodorizing effect to an odor of a substance or a composition to which application is intended and thereby carrying out screening. So far, in the case of the masking effect and the deodorizing effect to such odors being evaluated, it has been general to perform by using human bodies. However, in the case of the human body being used to evaluate, there are problems in that in addition to a large strain being forced on examinees, the examinees who take part in the evaluation can be secured with difficulty and furthermore from a human physiological viewpoint there is no warranty of the same phenomenon being always obtained. Furthermore, in the case of the evaluation being carried out with novel compounds and compositions, there is also a problem of safety. At this time, if a pseudo odor composition could be used in the evaluation, the novel compounds and compositions would be evaluated without paying attention to the problems as mentioned above.

From such viewpoints, pseudo human body odors for evaluating with simplicity the masking, harmonizing and deodorizing effect of the odor to particular odors such as the sweat odor, the armpit odor and so on have been looked for, hitherto, various human body odor components of such as a mouth odor, a scalp odor, an armpit odor, a foot odor, a sweat odor and so on have been studied. (see, for instance, Journal of Chemical Ecology, (USA) 1991,17 (7), pp. 1469 to 1492; Head Office of 46th TEAC Meeting (hosted by Chemical Society of Japan), 46th Discussion on perfume/Terpene and essential oil chemical, contents of lecture, 2002, pp. 124 to 126 ; and European Patent Application Laid-open No. 1,258, 531). WO 02/085294 A relates to the use of chemicals responsible for body odour in the search for suitable deodorants. Although the claims of this document specific butyric, propionic, isovaleric, hexanoic and 3-methyl-2-hexenoic acids or mixtures thereof for this purpose, only the use of isovaleric acid is exemplified. The invention differs from WO02/085294A in that pseudo body odour compositions including the carboxylic acid constituents (A) and (B) along with the aldehyde constituent (C) are employed for this purpose, whereby constituent (A) can be 3-methyl-2-hexenoic acid and the definition of constituent (B) includes butyric, propionic and hexanoic acis. When a pseudo body odor composition very close to an actual body odor can be obtained, it is obvious that while studying, as mentioned above, the safety of the novel substance or composition to human bodies on one hand, on the other hand, in parallel therewith, irrespective of the safety, the masking, harmonizing or deodorizing effect of the substance or composition to the armpit odor resultantly the sweat odor can be accurately, simply and efficiently evaluated, and thereby the substances and compositions that have the effect can be efficiently subjected to the screening.

On the basis of studies on these body odor components, studies for preparing model odors and proposals of model odors have been forwarded. Among the pseudo human body odors, as to the scalp odor, a model odor for evaluation comprising squalene, cholesterols and fatty acids as main components is proposed (JP-A Nos. 2001-220593 and 2001- 271088). On the other hand, as to the sweat odor, there have been mainly studied components that are generated when a sebaceous matter and higher fatty acids are oxidatively decomposed owing to indigenous bacteria in skin. However a pseudo odor more close to actual sweat odor, in particular, a peculiar odor secreted from the apocrine gland of such as armpits has not yet been obtained. The apocrine gland is present also in eyelids, external auditory meatus, the surroundings of anus, surroundings of nipple and so on other than the armpits.

On the other hand, in the deodorant products, various kinds of perfume compositions are blended ; however, it is a present state that as to perfume materials that can effectively mask the odor of armpits, the perfume materials having characteristics with sufficient satisfaction are not yet provided.

### Disclosure of Invention

In view of these circumstances, the present inventors analyzed components obtained by solvent extraction from a human armpit portion by use of gas chromatography, Olfactometry, mass spectrometry and so on and found this time a peak showing an odor characteristic to the armpit odor. Furthermore, by means of mass spectrometry analysis and chemical synthesis, the components were determined. So far, as the components of the sweat odor, various kinds of carboxylic acids including 3-methyl-2-hexenoic acid and acetic acid were reported. In addition, as a result of the research due to the present inventors, it was newly found that 3-hydroxy-3-methylhexanoic acid is contained in the components of the armpit odor and an enantiomer ratio of the hydroxycarboxylic acid, that is, a ratio of R-body to S-body of the enantiomers is 1: 3. The odor of the compound was an odor that suggests the armpit odor different from that of the so far known 3-methyl-2-hexenoic acid. As to the odor of the hydroxycarboxylic acid, a method of masking it with an odorous component has not yet been reported.

Accordingly, an object of the present invention is to provide a pseudo body odor composition that is more close to an actual sweat odor, in particular to a peculiar odor excreted from an apocrine gland and that allows evaluating the masking accurately, inhibition and so on of a sweat odor and an armpit odor; and a method of evaluating the effects of masking, harmonizing or deodorizing the sweat odor such as the armpit odor by use of the composition.

The present inventors newly built pseudo body odor compositions on the basis of the results of the analysis of an armpit odor and carried out the screening to many single perfumes with the new pseudo body odor compositions. As a result, it was found that some kinds of perfumes can efficiently mask and/or harmonize the acid odor such as the sweat odor and the armpit odor, that is, the acid odor selected from the aforementioned hydroxyalkynyl carboxylic acids having 5 to 8 carbon atoms and alkenyl carboxylic acids having 5 to 8 carbon atoms and one double bond, and thereby the present invention was achieved.

That is, the present invention relates to pseudo body odor compositions according to the following items [1] to [4] and a method of evaluating a masking, harmonizing or deodorizing effect of the sweat odor according to the following item [5].
[1] A pseudo body odor composition, comprising:
   (A) at lest one kind selected from (A-1) hydroxy alkynyl carboxylic acids having 5 to 8 carbon atoms and (A- 2) alkenyl carboxylic acids having 5 to 8 carbon atoms and one double bond ;
   (B) at least one kind selected from fatty acids having 2 to 24 carbon atoms ; and
   (C) at least one kind selected from aldehydes having 2 to 13 carbon atoms.
[2] The pseudo body odor composition set forth in the item [1] above, wherein the component (A) comprises:
   (A-1) at least one kind selected from hydroxyalkynyl carboxylic acids having 5 to 8 carbon atoms ; and
   (A-2) at least one kind selected from alkenyl carboxylic acids having 5 to 8 carbon atoms and one double bond.
[3] The pseudo body odor composition set forth in the item [1] or [2] above, wherein the component (A-1) is at least one kind selected from a racemic body and an optically active body of 3-hydroxy-3-methylhexanoic acid.
[4] The pseudo body odor composition set forth in any one of the items [1] to [3] above, wherein the component (A-2) is at least one kind selected from a cis-isomer and a trans-isomer of 3-methyl-2-hexenoic acid.
[5] A method of evaluating an effect of masking, harmonizing or deodorizing a sweat odor, characterized by using the pseudo body odor composition set forth in any one of the items [1] to [4].

### Brief Description of the Drawings

Fig. 1 is a gas chromatogram of an extract extracted with ether from absorbent cotton that was used to wipe a portion of armpit.
Fig. 2 is an example of a synthesis path diagram of an R-enantiomer and an S-enantiomer of 3-hydroxy-3methylhexanoic acid.
Fig. 3 is a gas chromatogram diagram showing by comparing results of identification in which a particular component of an extract extracted with ether from the absorbent cotton that was used to wipe a portion of armpit and methyl esters of synthesized 3-hydroxy-3-methylhexanoic acid (R-enantiomer and S-enantiomer) were used.

### Modes for Carrying Out the Invention

In advance of the explanation of the present invention, firstly analysis of the armpit odor and identification of components will be explained.

### [Component analysis of armpit odor and method of analyzing and identifying a characteristic peak]

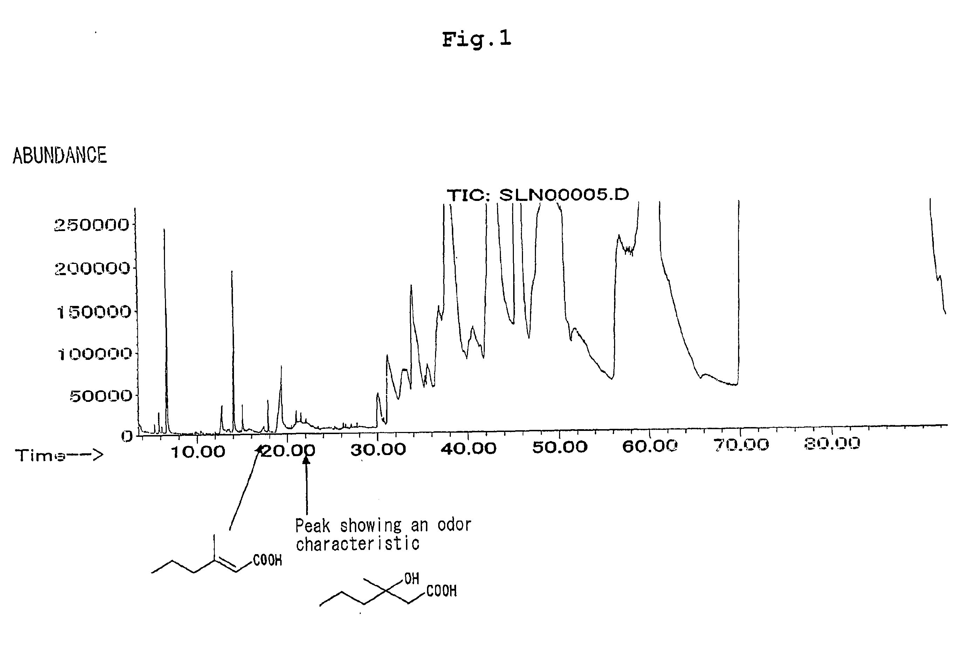

After the absorbent cotton that was used to wipe the armpit portion was extracted with ether, a solvent was concentrated and thereby an extracted sample was prepared. The sample was analyzed by use of a sniffing gas chromatography, a mass spectrometry and so on. Fig. 1 shows a gas chromatogram of an extract that was obtained by extracting with ether the absorbent cotton used to wipe the armpit portion. According to present inventor's analysis by use of the sniffing gas chromatography, a peak showing a characteristic odor was found.

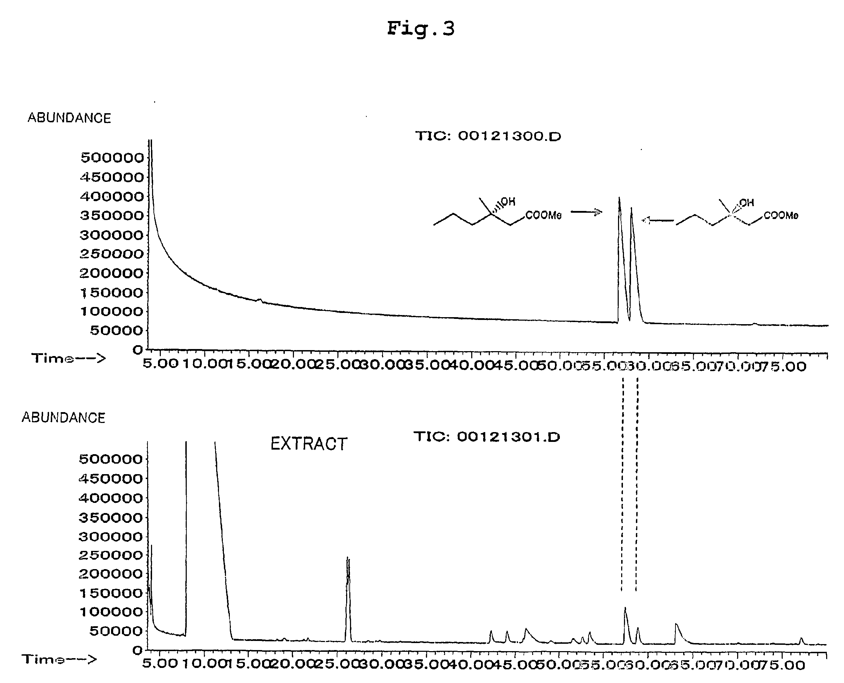

Further, from the mass spectrometry analysis of the peak showing the characteristic odor and a comparison with a compound obtained by chemical synthesis, it was clarified that a component of the peak showing the characteristic odor is 3-hydroxy-3-methylhexanoic acid, and furthermore in the hydroxycarboxylic acid a ratio of R-body to S-body that is an enantiomer ratio of both enantiomers is 1 : 3 (see Fig. 3).

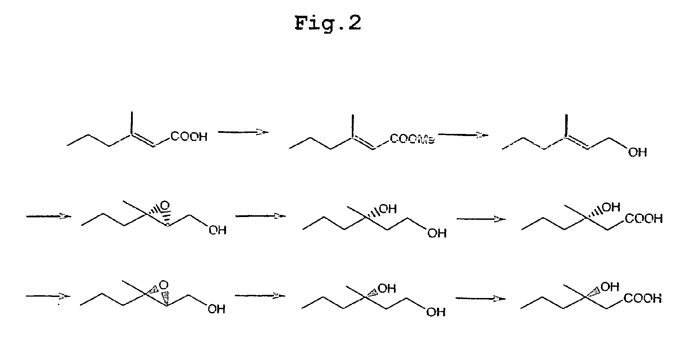

In addition, the R-enantiomer and the S-enantiomer of 3-hydroxy-3-methylhexanoic acid can be synthesized through a path shown in Fig. 2. The method of synthesis thereof can be referred to, for instance, JP-A No. 10-25265, J. Org. Chem. (USA), 1983, vol. 48, No. 22, pp. 3960 to 3966, and Tetrahedron Asymmetry (USA), 1996, vol. 7, No. 7, pp. 2023 to 2028.

In Table 1 below, odor quality of 3-hydroxy-3-methylhexanoic acid that was this time found and is an odor component that shows the odor characteristic to the sweat odor and that of 3-methyl-2-hexenoic acid that has been so far known as an odor component of the armpit odor are shown.

**Table 1**

| | Perfume quality |
|---|---|
| 3-hydroxy-3-methylhexanoic acid (racemic body) | Cumin spice like odor like an armpit odor weaker than an S-body |
| (R) -3-hydroxy-3-methylhexanoic acid | Cumin spice like odor weaker than racemic body |
| (S) -3-hydroxy-3-methylhexanoic acid | Armpit odor like and cumin spice like odor |
| 3-hydroxy-3-methylhexanoic acid, (R)-body/ (S)-body = 1/3 | Strong armpit like and cumin spice like odor |
| trans-3-methyl-2-hexenoic acid | Acidic odor |
| cis-3-methyl-2-hexenoic acid | Acidic odor |
| 3-methyl-2-hexenoic acid (trans-isomer/cis-isomer = 1/1) | Acidic odor stronger than cis-isomer |

### (1) Pseudo body odor composition

In the present invention, on the basis of the above results, considerations and evaluations were carried out, further the reconstruction of the compositions were performed, and thereby the present inventive pseudo body odor compositions that are more close to an actual odor of armpits were obtained.

Carboxylic acids of the above component (A) in the pseudo body odor compositions of the invention are important in the reproduction of an "odor peculiar to the apocrine gland" that is a peculiar odor due to the apocrine gland excretions in the sweat odors. The "odor peculiar to apocrine gland" denotes one that is displayed with the apocrine gland excretions as a cause material and has the nature of strongly smelling as the body odor (odor of sweat, odor of armpits). The odor peculiar to the apocrine gland strongly and instantaneously smells particularly in crowded trains and at the time when a person moved from one room to another room and gives more unpleasant sensation to others. When the odor peculiar to the apocrine gland is not taken into consideration, the preparation of the odor close to the sweat odor and the armpit odor in particular cannot be achieved.

The component (A) of the pseudo body odor composition according to the invention comprises at least one kind of hydroxyalkynyl carboxylic acids having 5 to 8 carbon atoms that is the component (A-1) and/or at least one kind of alkenyl carboxylic acids having 5 to 8 carbon atoms and one double bond that is the component (A-2).

As the hydroxyalkynyl carboxylic acids having 5 to 8 carbon atoms of the component (A-1), there are illustrated, for instance, 3-hydroxy-3-methylpentanoic acid, 3-hydroxy- 3-methylhexanoic acid and so on, preferably a racemic body and an optically active substance of 3-hydroxy-3-methylhexanoic acid, but the hydroxyalkynyl carboxylic acids are not restricted to ones described before. Further, in the 3-hydroxy-3-methylhexanoic acids, a ratio of R-body to S-body that is an enantiomer ratio is more preferably in the range of about from 1.5 : 1 to 1 : 20, furthermore preferably in the range of from about 1 : 1 to about 1: 5. The component (A-1) can be contained in the range of about from 0.1 to 50% by weight in a whole composition, preferably in the range of from about 1 to 30% by weight, and more preferably in the range of from 5 to 20% by weight.

On the other hand, as alkenyl carboxylic acids having 5 to 8 carbon atoms and one double bond of the component (A-2), there are illustrated, for instance, 3-methyl-2-pentenoic acids, 3-methyl-2-hexenoic acids, 3-methyl-2- heptenoic acids and so on. In addition, preferable examples of the alkenyl carboxylic acids include a cis- isomer and a trans-isomer of 3-methyl-2-hexenoic acid and more preferable examples thereof include ones in which a ratio of cis-isomer to trans-isomer that is a geometrical isomer ratio of 3-methyl-2-hexenoic acid is in the range of from about 1: 1 to about 1: 10. But the alkenyl carboxylic acids are not restricted to particular ones described above. The component (A-2) can be contained in the range of about from 0.1 to 50% by weight in a whole composition, preferably in the range of about from 1 to 30% by weight, and more preferably in the range of from 5 to 20% by weight.

Further, in the present invention, the component (A-1) and the component (A-2) are preferably used together. At this time, as a combination of the component (A-1) and the component (A-2), a combination of, for instance, 3-hydroxy-3-methylhexanoic acid in which a ratio of optically active R-enantiomer to S-enantiomer of 3-hydroxy-3-methylhexanoic acid is in the range of from 1 : 1 to 1: 3 and 3-methyl-2- hexenoic acid in which a ratio of cis-isomer to trans- isomer is in the range of from 1: 1 to 1: 10 and more preferably in the range of from 1: 1 to 1: 3 can be illustrated. In the case of the component (A-1) and the component (A-2) being used together, a ratio thereof, in terms of (A-1) to (A-2), is in the range of from 1: 1 to 10: 1 and particularly preferably in the range of from 1: 1 to 3: 1.

In the present invention, as the fatty acids having 2 to 24 carbon atoms shown as the components (B), straight chain or branched, saturated or unsaturated fatty acids that may have a hydroxyl group such as acetic acid, propionic acid, butanoic acid, isobutanoic acid, n-valeric acid, isovaleric acid, hexanoic acid, heptanoic acid, octanoic acid, nonanoic acid, decanoic acid, dodecanoic acid, tridecanoic acid, myristic acid, pentadecanoic acid, palmitic acid, palmitoleic acid, heptadecanoic acid, stearic acid, oleic acid, linoleic acid, glycolic acid, lactic acid, 3-hydroxybutanoic acid and so on can be illustrated. Among these, any one of acetic acid and propionic acid is an important component in reproducing an acidic odor of actual sweat odor and armpit odor, and when another fatty acid is added thereto, preferable sweat odor and armpit odor can be prepared. Here, as other fatty acids, fatty acids having 4 to 18 carbon atoms such as butanoic acid, isobutanoic acid, valeric acid, isovaleric acid, hexanoic acid, heptanoic acid, octanoic acid, nonanoic acid, decanoic acid, dodecanoic acid, tridecanoic acid, myristic acid, pentadecanoic acid, palmitic acid, palmitoleic acid, heptadecanoic acid, stearic acid, oleic acid, linoleic acid and so on can be preferably illustrated. Among these, fatty acids having 8 to 18 carbon atoms are important in reproducing a greasy odor of the sweat odors including the armpit odor.

A blending ratio of the fatty acids having 2 to 24 carbon atoms of the component (B) is, for acetic acid or propionic acid, preferably in the range of about from 0.001 to 50% by weight in a whole composition, more preferably in the range of about from 0.1 to 20% by weight and furthermore preferably in the range of about from 5 to 15% by weight; and, for fatty acids having 4 to 24 carbon atoms, the blending ratio is preferably in the range of about from 0.001 to 80% by weight in a whole composition, more preferably in the range of about from 0.1 to 70% by weight and furthermore preferably in the range of from 1 to 50% by weight.

In the invention, as aldehydes having 2 to 13 carbon atoms shown as the component (C), there are illustrated straight chain or branched, saturated or unsaturated aldehydes such as acetaldehyde, propionaldehyde, butylaldehyde, valeraldehyde, isovaleraldehyde, hexylaldehyde, heptylaldehyde, octylaldehyde, nonylaldehyde, decylaldehyde, undecylaldehyde, dodecylaldehyde, tridecylaldehyde, tetradecylaldehyde, hexadecylaldehyde, 3- hexenal, 4-heptenal, 2-nonenal, 4-decenal, undecylenic aldehyde, 2,6-nonadienal, decadienal, methyl octyl acetaldehyde, methyl nonyl acetaldehyde and so on. Among these, saturated aldehydes having 6 to 12 carbon atoms are important in reproducing the greasy odor of the sweat odor and the armpit odor. A compounding ratio of the aldehyde having 2 to 13 carbon atoms of the component (C) is, in the whole composition, preferably in the range of about from 0.001 to 50% by weight, more preferably in the range of about from 0.01 to 10% by weight, and furthermore preferably in the range of from 0.1 to 5% by weight.

In order to adjust a delicate quality and concentration of the body odor such as the odor of sweat and the odor of armpits, other than the components (A) to (C), squalene, squalane, cholesterols or their derivates, amino acids, glycerides, straight chain or branched fatty acid esters having 2 to 24 carbon atoms, volatile nitrogen-containing compounds, volatile sulfur-containing compounds and so on may be blended to the pseudo body odor compositions of the invention. These can be added in the range of about from 0.00001 to 10000 parts by weight relative to one part by weight of the components (A) to (C).

In these compounding compounds other than the components (A) to (C), as the cholesterol derivatives, there are illustrated cholesterol esters and so on of straight chain or branched saturated or unsaturated fatty acids having 11 to 16 carbon atoms such as cholesterol ester of undecanoic acid, cholesterol ester of dodecanoic acid, cholesterol ester of tridecanoic acid, cholesterol ester of tetradecanoic acid, cholesterol ester of pentadecanoic acid, cholesterol ester of palmitic acid, cholesterol ester of palmitoleic acid and so on. Further, as amino acids, there are illustrated proline, proline- containing peptides, leucine, phenylalanine, serine, threonine, citrunine, valine, lysine, arginine, amino acids formed by decomposition of proteins owing to a protease and so on. As the glycerides, there are illustrated glycerides of straight chain or branched, saturated or unsaturated fatty acids having 2 to 24 carbon atoms. such as decanoic acid, undecanoic acid, dodecanoic acid, tridecanoic acid, tetradecanoic acid, myristic acid, pentadecanoic acid, palmitic acid, palmitoleic acid and so on. The glycerides include all of mono-, di-and tri-fatty acid glycerides. However, triglycerides, in particular, triglycerides of straight chain or branched saturated or unsaturated fatty acid having 16 carbon atoms such as palmitic acid triglyceride, palmitoleic acid triglyceride and so on are preferred. As the fatty acid esters, there are illustrated, for instance, hexyl ester of lauric acid, heptyl ester of lauric acid, octyl ester of lauric acid, nonyl ester of lauric acid, hexyl ester of palmitic acid, heptyl ester of palmitic acid, octyl ester of palmitic acid, nonyl ester of palmitic acid, hexyl ester of palmitoleic acid and so on. Further, as the volatile nitrogen-containing compounds and volatile sulfur-containing compounds, there are illustrated ammonia, trimethylamine, tributamine, hydrogen sulfide, methyl mercaptan and so on.

Furthermore, in order to adjust a concentration of the pseudo body odor composition according to the invention, a solvent (solubilizing agent) may be compounded into the composition, this being important in recreating an intensity of an actual body odor. In a chemical constitution of sweat, it is known that a main component other than water is lactic acid (see, for instance, Boukin Boubai, 1985, Vol. 13, No. 8, pp. 363 to 372), and since lactic acid does not affect on the odor of the sweat odor (armpit odor) and works as a solubilizing agent, the use thereof as the solubilizing agent of the pseudo body odor composition according to the invention is preferable in recreating the component of actual sweat odor and armpit odor. In addition, as one that has the same characteristics as lactic acid, fatty acids that have 2 to 4 carbon atoms and a hydroxy group such as glycolic acid, 3-hydroxy butanoic acid and so on can be cited, and these are also preferably used as the solubilizing agent for the pseudo body odor composition of the invention.

As the solubilizing agent other than the above solubilizing agent, any one that does not affect on the odor such as the sweat odor and the armpit odor can be used without restricting to particular one. As such other solubilizing agents other than the above solubilizing agents, there are preferably illustrated, for example, ethanol, 3-methoxy-3-methyl butanol, propylene glycol, dipropylene glycol, diethyl phthalate, triethyl citrate, benzyl benzoate, liquid paraffin and so on. Among these other solubilizing agents, when the viscosity and the solubilization are taken into consideration in view of using the composition for evaluation, ethanol, 3-methoxy-3-methyl butanol, dipropylene glycol, diethyl phthalate, triethyl citrate, benzyl benzoate and so on are preferred, and from the viewpoint of the odorlessness, dipropylene glycol, diethyl phthalate, triethyl citrate and benzyl benzoate are preferred. The solubilizing agent can be preferably compounded by about 0. 001 to 10000 parts by weight relative to one part by weight of a total amount of components (A) to (C).

Thus prepared pseudo body odor composition is close to actual sweat odor, armpit odor and so on. Therefore ; by use of the composition, accurate and efficient evaluations of the masking capability, the harmonizing effect or the deodorizing effect to the sweat odor, armpit odor and so on can be made. As a result, substances that have the masking capability, the harmonizing effect or the deodorizing effect to the odor of the sweat odor, armpit odor and so on can be efficiently screened. Thus, the pseudo body odor compositions and the evaluation methods according to the invention are useful in developing masking agents of odor, deodorants and odor inhibitors and further various kinds of body deodorants, various kinds of detergents for body and various kinds of detergents for clothing to which these masking agents, deodorants or odor inhibitors are blended.

When the pseudo body odor composition for use in evaluation of the invention are used, the evaluation of the masking capability, the harmonizing effect or the deodorizing effect to the body odor, in particular, the sweat odor, the armpit odor and so on caused by human sweat can be easily performed by, for instance, adding a specimen to the pseudo body odor composition for use in evaluation of the invention or bringing a specimen into contact with the inventive composition, followed by smelling and comparing the odor with a odor of a body odor composition of the invention. As a specific method, a cup method may be used. In the evaluation of the odor, it is preferable to carry out by 1 to 20 expert panelists and general panelists. Further, the evaluation is preferably carried out by a 2 to 7 grades evaluation, and more preferably a 3 to 5 grades evaluation by expert panelists.

By use of the pseudo body odor composition for use in evaluation according to the invention, effects of masking, harmonizing or deodorizing the body odor, in particular, the sweat odor, the armpit odor and so on caused by human sweat can be easily evaluated, and substances having these effects can be accurately and efficiently screened. In addition, while sufficiently studying the safety on one hand, on the other hand, in parallel with the study of the safety, functional evaluation can be performed. Accordingly, efficient product development can be realized.

Furthermore, the perfume compositions for inhibiting a body odor can be put in prescriptions for preparing perfumeries and can be prepared into desired modes and dosage forms. As the modes and the dosage forms of thus prepared perfumeries, there are illustrated a perfume, an eau de cologne, an eau de toilette, a deodorant, soap, body soap, a shampoo, a detergent and so on as examples. In these cases, a compounding amount of the perfume composition according to the invention in the perfumery is not particularly restricted but is preferably in the range of about from 0.001 to 20.0% by weight relative to the whole amount of the perfumery.

The perfume compositions for inhibiting a body odor of the invention are compounded into various kinds of deodorants and the external preparation compositions, and exhibit excellent masking effect and/or harmonizing effect for the acid odors selected from hydroxyalkynyl carboxylic acids having 5 to 8 carbon atoms and alkenyl carboxylic acids having 5 to 8 carbon atoms and one double bond that are emitted from the sweat odor, in particular, the armpit odor. The deodorants and the external preparations wherein the perfume compositions for inhibiting a body odor of the invention are compounded may have the so-called deodorant effect as one of objects thereof. That is, the perfume compositions for inhibiting a body odor of the invention include all of so-called deodorants, external preparations such as skin external preparations and so on, wash finishing agents or coating agents for clothing as means for preventing emission of the acid odors that are liable to adhere to the clothing and so on or means for preventing adhesion of the acid odors emitted from human bodies to the clothing, perfumeries, and so on. That is, deodorants and the external preparation compositions for inhibiting a body.

### Examples

In the following, the present invention will be more specifically explained by reference to Examples. However, the present invention is not restricted to the following Examples and Comparative Examples.

### Examples 1 to 7 and Comparative Example 1

Pseudo sweat odor compositions for use in evaluation which have constitutions shown in Tables 2 and 3 were prepared according to a standard method and evaluated synthetically on the basis of the following evaluation methods. Results thereof are together shown in Tables 2 and 3.

### [Evaluation method]

An odor evaluation was carried out, in particular, on sweat odor likeliness and armpit odor likeliness of an odor in a cup (internal volume of about 500 ml), which a 2 cm x 2 cm filter paper with 200 µl of a pseudo body odor composition was put in, according to the following criteria by four expert panelists. As a result, pseudo body odor compositions that were evaluated as less than 3 points in average point of the four panelists were judged appropriate as pseudo body odor compositions for use in evaluation.

### [Evaluation criteria]

1: Obviously senses the body odor likeliness, in particular, as a sweat odor and armpit odor.
2: Sufficiently senses the body odor likeliness as a sweat odor and armpit odor.
3: Slightly senses the body odor likeliness as a sweat odor and armpit odor.
4: Hardly senses the body odor likeliness as a sweat odor and armpit odor.

**Table 2**

| Constitution (% by weight) | Example 1 | Example 2 | Example 3 | Example 4 |
|---|---|---|---|---|
| 3-Hydroxy-3-methylhexanoic acid (racemic body) | 13.00 | 5.00 | 5.00 | 0.00 |
| 3-Hydroxy-3-methylhexanoic acid (enantiomer ratio: R/S = 1/3) | 0.00 | 0.00 | 0.00 | 13.00 |
| 3-Methyl-2-hexenoic acid (trans isomer/cis isomer 1/1) | 5.00 | 13.00 | 5.00 | 5.00 |
| Acetic acid | 11.85 | 11.85 | 12.60 | 11.85 |
| Isobutanoic acid | 3.95 | 3.95 | 4.20 | 3.95 |
| Butanoic acid | 0.79 | 0.79 | 0.84 | 0.79 |
| Isovaleric acid | 3.95 | 3.95 | 4.20 | 3.95 |
| Hexanoic acid | 0.79 | 0.79 | 0.84 | 0.79 |
| Heptanoic acid | 0.79 | 0.79 | 0.84 | 0.79 |
| Octanoic acid | 0.79 | 0.79 | 0.84 | 0.79 |
| Nonanoic acid | 0.79 | 0.79 | 0.84 | 0.79 |
| Lactic acid | 55.30 | 55.30 | 58.80 | 55.30 |
| Hexyl aldehyde | 0.60 | 0.60 | 1.20 | 0.60 |
| Octyl aldehyde | 0.36 | 0.36 | 0.72 | 0.36 |
| Nonyl aldehyde | 0.09 | 0.09 | 0.18 | 0.09 |
| Decyl aldehyde | 0.90 | 0.90 | 1.80 | 0.90 |
| Undecyl aldehyde | 0.60 | 0.60 | 1.20 | 0.60 |
| Dodecyl aldehyde | 0.23 | 0.23 | 0.45 | 0.23 |
| Benzyl benzoate | 0.23 | 0.23 | 0.45 | 0.23 |
| Triethyl citrate | 0.00 | 0.00 | 0.00 | 5.00 |
| Total | 100.00 | 100.00 | 100.00 | 100.00 |
| Body odor (sweat odor and armpit odor) likeliness | 1.0 | 2.0 | 2.0 | 1.0 |

**Table 3**

| Constituition (% by weight) | Example 5 | Example 6 | Example 7 | Comparative Example 1 |
|---|---|---|---|---|
| 3-Hydroxy-3-methylhexanoic acid (racemic body) | 13.00 | 0.00 | 0.00 | 0.00 |
| 3-Hydroxy-3-methylhexanoic acid (enantiomer ratio: R/S = 1/3) | 0.00 | 0.00 | 13.00 | 0.00 |
| 3-Methyl-2-heenoic acid (trans isomer/cis isomer = 1/1) | 0.00 | 13.00 | 0.00 | 0.00 |
| Acetic acid | 11.85 | 11.85 | 11.85 | 11.85 |
| Isobutanoic acid | 3.95 | 3.95 | 3.95 | 3.95 |
| Butanoic acid | 0.79 | 0.79 | 0.79 | 0.79 |
| Isovaleric acid | 3.95 | 3.95 | 3.95 | 3.95 |
| Hexanoic acid | 0.79 | 0.79 | 0.79 | 0.79 |
| Heptanoic acid | 0.79 | 0.79 | 0.79 | 0.79 |
| Octanoic acid | 0.79 | 0.79 | 0.79 | 0.79 |
| Nonanoic acid | 0.79 | 0.79 | 0.79 | 0.79 |
| Lactic acid | 55.30 | 55.30 | 55.30 | 55.30 |
| Hexyl aldehyde | 0.60 | 0.60 | 0.60 | 0.60 |
| Octyl aldehyde | 0.36 | 0.36 | 0.36 | 0.36 |
| Nonyl aldehyde | 0.09 | 0.09 | 0.09 | 0.09 |
| Decyl aldehyde | 0.90 | 0.90 | 0.90 | 0.90 |
| Undecyl aldehyde | 0.60 | 0.60 | 0.60 | 0.60 |
| Dodecyl aldehyde | 0.23 | 0.23 | 0.23 | 0.23 |
| Benzyl benzoate | 0.23 | 0.23 | 0.23 | 0.23 |
| Triethyl citrate | 5.00 | 5.00 | 5.00 | 18.00 |
| Total | 100.00 | 100.00 | 100.00 | 100.00 |
| Body odor (sweat odor and armpit odor) likeliness | 2.5 | 2.0 | 2.0 | 3.5 |

As obvious from results shown in Tables 2 and 3, Examples 1 to 7 that are present inventive products all were excellent as pseudo body odor compositions for use in evaluation. In addition, as for 3-hydroxy-3-methylhexanoic acid, as obvious from results of evaluation according to Examples 5 in which racemic body of 3-hydroxy-3-methylhexanoic acid was used and Examples 7 in which 3- hydroxy-3-methylhexanoic acid having the enantiomer ratio: R/S = 1/3 was used, it is found that the evaluation of the body odor likeliness of 3-hydroxy-3-methylhexanoic acid having the enantiomer ratio: R/S = 1/3 is higher than that of the racemic body, that is, 3-hydroxy-3-methylhexanoic acid having the enantiomer ratio: R/S = 1/3 is excellent than the racemic body.

### Example 8

Using compounds shown in Table 4 and a perfume composition A, suppression tests to evaluate the masking effect and the harmonizing effect of the body odor (sweat odor and armpit odor) were carried out according to a procedure of the following Experimental Example 1, test subjects were evaluated according to the following evaluation criteria, and the evaluations were shown as evaluated values. Results thereof are together shown in Table 4. In addition, a constitution of the perfume composition A in Table 4 is shown in Table 5.

### Experimental Example 1

Both a 2 cm x 2 cm filter paper with 200 µl of a pseudo body odor composition obtained according to Example 4 and having a constituent shown in Table 6 below and a 2 cm x 2 cm filer paper with 10 µl of a perfume were put into a cup having an internal volume of about 500 ml, followed by sealing with an aluminum foil. After 30 minutes, a hole of about 1 cm in diameter was formed in the aluminum foil and the investigations of masking effect and the harmonizing effect were carried out according to following evaluation criteria by four expert panelists.

### [Evaluation criteria of the masking effect]

3: Excellent in the masking.
2: Substantially excellent in the masking.
1: Slightly sensible of the body odor (sweat odor and armpit odor).
0: Sensible of the body odor (sweat odor and armpit odor) (insufficient in the masking)

### [Evaluation criteria of the harmonizing effect]

1: In harmony.
0: Indefinite.
-1 : Not in harmony.

**Table 4**

| | Masking | Harmonizing |
|---|---|---|
| 2-Acetyl-2,3,8,8-tetramethyl octarin | 1 | -1 |
| β-Damascone | 2 | 1 |
| γ-Methylionone | 2 | 1 |
| Ethyl linalool | 1.5 | 0 |
| Eugenol | 1.5 | 0 |
| Coumarin | 2.5 | 1 |
| Geraniol | 2 | 1 |
| Phenylethyl acetate | 2.5 | 1 |
| cis-3-Hexenyl salicylate | 2 | 1 |
| cis-Jasmone | 1 | -1 |
| Citronellol | 2.5 | 1 |
| Phenylethyl alcohol | 2.5 | 1 |
| Phenyl ethyl dimethyl carbinol | 1.5 | -1 |
| Heliotropine | 1.5 | 0 |
| Benzaldehyde | 3 | 1 |
| Raspberry ketone | 0.5 | -1 |
| Linalool | 1 | 0 |
| Lemon oil | 3 | 1 |
| Rose oil | 2.5 | 1 |
| Rose oxide | 3 | 1 |
| Perfume composition A | 2.5 | 1 |

**Table 5**

| Constitution | Parts by weight |
|---|---|
| 2-Acetyl-2,3,8,8-tetramethyl octarin | 200 |
| γ-Methylionone | 5 |
| Methyl anthranilate | 4 |
| Coumarin | 5 |
| Geraniol | 80 |
| Trichloromethylphenylcarbinyl acetate | 20 |
| Phenylethyl acetate | 100 |
| cis-3-Hexenyl salicylate | 20 |
| cis-Jasmone | 1 |
| Citronellol | 50 |
| Farnesol | 50 |
| Phenylethyl alcohol | 90 |
| Phenyl ethyl dimethyl carbinol | 180 |
| Heliotropine | 100 |
| Linalool | 50 |
| Sandalwood oil | 15 |
| Bergamot oil | 10 |
| Rhyme oil | 10 |
| Lemon oil | 10 |
| Total | 1000 |

**Table 6**

| Constitution of pseudo body odor | % by weight |
|---|---|
| 3-Hydroxy-3-methylhexanoic acid (enantiomerratio: R/S = 1/3) | 13.00 |
| 3-Methyl-2-hexenoic acid (trans isomer/cis isomer = 1/1) | 5.0 |
| Acetic acid | 11.85 |
| Isobutanoic acid | 3.95 |
| Butanoic acid | 0.79 |
| Isovaleric acid | 3.95 |
| Hexanoic acid | 0.79 |
| Heptanoic acid | 0.79 |
| Octanoic acid | 0.79 |
| Nonanoic acid | 0.79 |
| Lactic acid | 55.30 |
| Hexyl aldehyde | 0.60 |
| Octyl aldehyde | 0.36 |
| Nonyl aldehyde | 0.09 |
| Decyl aldehyde | 0.90 |
| Undecyl aldehyde | 0.60 |
| Dodecyl aldehyde | 0.23 |
| Benzyl benzoate | 0.23 |
| Triethyl citrate | 5.00 |
| Total | 100.00 |

### Example 9

According to the procedure of the above Experimental Example 1, the suppression tests were conducted in the same manner as in Example 8 to evaluate the masking effect and the harmonizing effect of body odors (sweat odor and armpit odor) of many test subjects. On the basis of the results thereof, components of the perfume compositions for inhibiting a body odor of the invention may be selected.

The perfume compositions for deodorant of Example 16 and Comparative Example 8 were compounded according to the following example of prescription to prepare lotions that are external preparation composition.

## Claims

1. A pseudo body odor composition, comprising:
(A) at least one kind selected from (A-1) hydroxyalkynyl carboxylic acids having 5 to 8 carbon atoms and (A-2) alkenyl carboxylic acids having 5 to 8 carbon atoms and one double bond ;
(B) at least one kind selected from fatty acids having 2 to 24 carbon atoms; and
(C) at least one kind selected from aldehydes having 2 to 13 carbon atoms.

2. The pseudo body odor composition according to claim 1, wherein the component (A) includes
(A-1) at least one kind selected from hydroxyalkynyl carboxylic acids having 5 to 8 carbon atoms; and
(A-2) at least one kind selected from alkenyl carboxylic acids having 5 to 8 carbon atoms and one double bond.

3. The pseudo body odor composition according to claim 1 or 2, wherein the component (A-1) is at least one kind selected from a racemic body and an optically active substance of 3-hydroxy-3-methylhexanoic acid.

4. The pseudo body odor composition according to any one of claims 1 to 3, wherein the component (A-2) is at least one kind selected from a cis-isomer and a trans- isomer of 3-methyl-2-hexenoic acid.

5. A method of evaluating a masking, harmonizing or deodorizing effect to a sweat odor **characterized by** using the pseudo body odor composition according to any one of claims 1 to 4.

## Patentansprüche

1. Pseudokörpergeruchszusammensetzung, mit:
(A) mindestens einer Verbindung ausgewählt aus (A-1) Hydroxyalkynyl Carbonsäuren mit 5 bis 8 Kohlenstoffatomen und (A-2) Alkenyl Carbonsäuren mit 5 bis 8 Kohlenstoffatomen und einer Doppelbindung ;
(B) mindestens einer Verbindung ausgewählt aus Fettsäuren mit 2 bis 24 Kohlenstoffatomen; und
(C) mindestens einer Verbindung ausgewählt aus Aldehyden mit 2 bis 13 Kohlenstoffatomen.

2. Pseudokörpergeruchszusammensetzung nach Anspruch 1, wobei die Komponente (A) umfasst
(A-1) mindestens eine Verbindung ausgewählt aus Hydroxyalkynyl Carbonsäuren mit 5 bis 8 Kohlenstoffatomen; und
(A-2) mindestens eine Verbindung, ausgewählt aus Alkenyl Carbonsäuren mit 5 bis 8 Kohlenstoffatomen und einer Doppelbindung.

3. The Pseudokörpergeruchszusammensetzung nach Anspruch 1 oder 2, wobei die Komponente (A-1) mindestens eine Verbindung ausgewählt aus einem Racemischen Körper und einer optisch aktiven Substanz von 3-Hydroxy-3-methylhexansäure ist.

4. Pseudokörpergeruchszusammensetzung nach irgendeinem der Ansprüche 1 bis 3, wobei die Komponente (A-2) mindestens eine Verbindung ausgewählt aus einem cis-Isomeren und einem Trans- Isomeren von 3-Methyl-2-Hexansäure ist.

5. Verfahren zur Bewertung eines Maskierungs- , Harmonisierungs- oder Deodorant- Effekts auf einen Schweißgeruch, **gekennzeichnet durch** Verwendung der Pseudokörpergeruchszusammensetzung nach irgendeinem der Ansprüche 1 bis 4.

## Revendications

1. Préparation ayant une pseudo odeur corporelle, comprenant:
(A) au moins un type sélecté d'acides hydroxyalcynyle carboxyliques (A-1) ayant 5 à 8 atomes de carbone et d'acides alkényle carboxyliques (A-2) ayant 5 à 8 atomes de carbone et une double liaison;
(B) au moins un type sélecté d'acides gras ayant 2 à 24 atomes de carbone; et
(C) au moins un type sélecté d'aldéhydes ayant 2 à 13 atomes de carbone.

2. Préparation ayant une pseudo odeur corporelle selon la revendication 1, où le composant (A) comprend:
(A-1) au moins un type sélecté d'acides hydroxyalcynyle carboxyliques ayant 5 à 8 atomes de carbone; et
(A-2) au moins un type sélecté d'acides alkényle carboxyliques ayant 5 à 8 atomes de carbone et une double liaison.

3. Préparation ayant une pseudo odeur corporelle selon la revendication 1 ou 2, où le composant (A-1) est au moins un type sélecté d'un corps racémique et une substance optiquement active d'acide 3-hydroxy-3-méthylhéxanoique.

4. Préparation ayant une pseudo odeur corporelle selon l'une quelconque des revendications 1 à 3, où le composant (A-2) est au moins un type sélecté d'un cis- isomère et d'un trans-isomère d'acide 3-méthyle-2-hexénoïque.

5. Procédé d'évaluation un masquage, un effet d'harmonisation ou désodorisant à un odeur de sueur **caractérisé par** l'utilisation d'une préparation ayant une pseudo odeur corporelle selon l'une quelconque des revendications 1 à 4' .
